# FASCICULE DE BREVET EUROPEEN

(11) **EP 1 968 918 B1**
(45) Date de publication et mention de la délivrance du brevet: **08.11.2017**
(21) Numéro de dépôt: 06841967.0
(22) Date de dépôt: 14.12.2006
(51) Int. Cl.: C07C 2/36, C07C 11/107

(54) **PROCEDE POUR LA FABRICATION DE 2,3-DIMETHYLBUT-1-ENE**
VERFAHREN ZUR HERSTELLUNG VON 2,3-DIMETHYLBUT-1-EN
PROCESS FOR PREPARING 2,3-DIMETHYLBUT-1-ENE

(30) Priorité: 22.12.2005 FR 0513164
(43) Date de publication de la demande: 17.09.2008
(73) Titulaire: IFP Energies nouvelles, 92500 Rueil-Malmaison (FR)
(72) Inventeur: OLIVIER-BOURBIGOU, Hélène, F-69230 St Genis Laval (FR); PELLIER, Emmanuel, F-78320 Verriere (FR); FORESTIERE, Alain, F-69390 Vernaison (FR)
(86) Numéro de dépôt international: PCT/FR2006/002764
(87) Numéro de publication internationale: WO 2007/080287

(56) Documents cités:
- EP-A- 0 646 412
- EP-A- 1 122 230
- FR-A- 2 611 700
- US-A- 5 104 840

## Description

La présente invention concerne la fabrication du 2,3-diméthylbut-1-ène (2,3-DMB-1) par dimérisation du propylène.

Le brevet français FR-B-2 611 700 (au nom du même déposant) décrit l'utilisation de liquides. à caractère ionique formés d'halogénures d'aluminium et d'halogénures d'ammonium quaternaires comme solvants de complexes organométalliques du nickel, ces milieux étant décrits comme permettant la catalyse de dimérisation des oléfines. L'utilisation de ces milieux ioniques non miscibles avec les hydrocarbures aliphatiques, en particulier avec les produits issus de la dimérisation des oléfines, permet ainsi une meilleure utilisation des catalyseurs homogènes.

Le brevet US-A-5 104 840 décrit une composition liquide à caractère ionique (appelée "liquide ionique") résultant de la mise en contact d'au moins un halogénure d'ammonium quartenaire et/ou d'au moins un halogénure de phosphonium quaternaire avec au moins un dihalogénure d'alkylaluminium et éventuellement en outre un trihalogénure d'aluminium. Ce brevet montre l'utilisation de ces milieux comme solvants de complexes de métaux de transition, notamment des complexes du nickel ne contenant pas de liaison nickel-carbone, qui sont transformés ultérieurement en catalyseurs d'oligomérisation des oléfines. Il montre aussi que les catalyseurs de nickel les plus actifs et les plus stables sont obtenus dans des "liquides ioniques" constitués d'un équivalent molaire d'halogénure d'ammonium et/ou d'halogénure de phosphonium avec un équivalent et plus de trihalogénure d'aluminium, et éventuellement une quantité quelconque de dihalogénure d'alkylaluminium. Cette formulation s'est révélée particulièrement intéressante parce que les complexes du nickel qui y sont dissous présentent une activité catalytique élevée.

Par ailleurs, il a été vérifié que, dans les conditions décrites dans le brevet français FR-B-2 611 700, « l'effet phosphine » tel qu'il a été décrit par G. Wilke et al. dans Ind. Eng. Chem., 1970, 62, n°12, p. 34, et dans le brevet GB-B-1 058680, et qui traduit l'influence des substituants portés par l'atome de phosphore sur le mode d'enchaînement des molécules de propylène lors de sa dimérisation catalytique par le nickel, disparaissait rapidement au cours du temps, entraînant une diminution de la sélectivité. Ce phénomène inexpliqué a des conséquences néfastes puisqu'il ne permet pas d'obtenir les qualités de produit recherchées.

Il est décrit dans le brevet FR-B-2 710 280 du même déposant que l'addition d'un hydrocarbure aromatique au liquide ionique permet de pallier ce défaut et conduit à des catalyseurs dont l'activité est élevée et plus stable et dont la sélectivité en isomères les plus ramifiés, le 2,3-diméthylbut-1-ène (2,3-DMB-1) et le 2,3-diméthylbut-2-ène (2,3-DMB-2) est importante lorsqu'on applique ces conditions de dimérisation au propylène.

Comme le montre le Tableau 1 ci-dessous, les points d'ébullition du 2,3-DMB-1, du méthyl-4-pentène-1 (M4P1) et du méthyl-4-pentène-2 (M4P2), produits formés également lors de la dimérisation du propylène, diffèrent que de quelques degrés Celsius.

**Tableau 1 : Point d'ébullition des différents isomères formés par dimérisation du propylène**

| Isomère | Point d'ébullition (°C) |
|---|---|
| 4-Méthylpent-1-ène (M4P1) | 53,87 |
| 2,3-Diméthylbut-1-ène (2,3-DMB-1) | 55,2 |
| 4-Méthylpent-2-ène *cis* (M4P2c) | 56,4 |
| 4-Méthylpent-2-ène *trans* (M4P2t) | 58,61 |
| 2-Méthylpent-1-ène (M2P1) | 62,11 |
| Hexène-3 *trans* (H3t) | 66,45 |
| Hexène-2 *trans* (H2t) | 67,88 |
| 2-Méthylpent-2-ène (M2P2) | 67,31 |
| Hexène-2 *cis* (H2c) | 68,99 |
| 2,3-Diméthylbut-2-ène (2,3-DMB-2) | 73,21 |

L'obtention du 2,3-DMB-1 avec une bonne pureté peut s'avérer difficile si l'on utilise les techniques de distillation classiques. C'est la raison pour laquelle les brevets US-A-5 910 618 et US-A-4 835 328 décrivent un enchaînement d'étapes selon lesquelles le 2,3-DMB-1, produit avec un mélange d'isomères par dimérisation du propylène, est isomérisé en 2,3-DMB-2. Ce dernier, dont le point d'ébullition est plus élevé, est séparé plus facilement des autres isomères par distillation. Il peut éventuellement être reconverti ultérieurement en 2,3-DMB-1 par isomérisation, bien que l'équilibre thermodynamique soit défavorable à cette transformation, transformation qui est, de plus, altérée par des réactions de polymérisation qui en diminuent le rendement.

Il apparaît donc particulièrement intéressant de pouvoir produire du 2,3-DMB-1 par dimérisation du propylène avec une sélectivité suffisamment élevée pour qu'il puisse être raisonnablement séparé des autres isomères par distillation.

Le brevet US-B-6 388 160 décrit un procédé de fabrication de 2,3-DMB-1 et 2,3-DMB-2 par une suite d'étapes comprenant :
a) une dimérisation du propylène en un mélange riche en 2,3-DMB-1 (étape 1),
b) une distillation du 2,3-DMB-1 (étape 2),
c) une isomérisation du 2,3-DMB-1 contenu dans le résidu de distillation en 2,3-DMB-2 (étape 3) et
d) une séparation du 2,3-DMB-2 par distillation (étape 4).

La dimérisation du propylène décrite dans la première étape est réalisée par une composition catalytique comprenant au moins un composé du nickel, au moins un composé du phosphore trivalent, au moins un phénol halogéné ou un dérivé de l'isopropanol fluoré, au moins un trialkylaluminium et au moins un composé portant une fonction acide sulfonique. Cette composition catalytique est mise en oeuvre dans un solvant organique. Le catalyseur est neutralisé à la fin de la réaction. Le procédé opère donc à "catalyseur perdu". Ce dernier n'est pas régénéré ni recyclé. La sélectivité en dimères décrite dans l'Exemple 2 du brevet US-B-6 388 160 est de 74,5 % par rapport aux produits totaux formés. Les dimères contiennent 78,4 % de DMB-1. L'activité du système est décrite par le "DMB-1 TON", qui correspond au nombre de millimoles de DMB-1 produit divisé par le nombre de millimoles de nickel utilisé. Le TON est de 38 210.

Le document EP0646412A décrit une composition catalytique comportant un composé du nickel complexé ou mélangé avec une phosphine tertiaire, dissous dans un milieu non aqueux résultant de la mise en contact d'un halogénure d'aluminium avec un halogénure d'ammonium quaternaire et/ou un halogénure de phosphonium quaternaire, et un hydrocarbure aromatique, mais ne pas fait mention de l'utilisation d'un hydrocarbure aromatique consistant au moins en partie en du mésitylène.

Le document EP1122230A décrit un procédé de dimérisation sélective du propylène en dimères ramifiés, dans lequel on met en contact le propylène avec une composition catalytique comprenant un composé du nickel mélangé ou complexé avec une phosphine tertiaire fonctionnalisée, dans un milieu formé d'un mélange d'un halogénure d'ammonium et/ou de phosphonium quaternaire avec un halogénure d'aluminium, mais ne pas fait mention de l'utilisation d'un hydrocarbure aromatique consistant au moins en partie en du mésitylène.

Il a maintenant été trouvé, et c'est l'objet de la présente invention, qu'il était possible de produire sélectivement du 2,3-DMB-1 en mettant du propylène en contact avec une composition catalytique comprenant au moins un composé du nickel mélangé ou complexé avec au moins une phosphine tertiaire, au moins un hydrocarbure aromatique consistant au moins en partie en du mésitylène (triméthyl-1,3,5-benzène), au moins un milieu non-aqueux à caractère ionique résultant du mélange d'un halogénure d'ammonium quaternaire ou d'halogénure de phosphonium quaternaire avec un halogénure d'aluminium et éventuellement au moins un composé organométallique de l'aluminium.

Plus précisément, l'utilisation d'un hydrocarbure aromatique constitué au moins en partie de mésitylène permet de maintenir une sélectivité en 2,3-DMB-1 élevée et stable dans le temps, ce qui permet de séparer le 2,3-DMB-1 des autres isomères par distillation.

La dissolution du composé du nickel, de la phosphine et de l'hydrocarbure aromatique dans un milieu liquide à caractère ionique, très peu miscible avec les produits formés, permet une séparation facile, par simple décantation, du catalyseur et du liquide ionique des produits. Le liquide ionique contenant le catalyseur peut ainsi être recyclé et réutilisé dans un processus en continu ou en discontinu.

Ainsi, le procédé de fabrication du 2,3-diméthylbut-1-ène (2,3-DMB-1) selon l'invention comprend :
- une étape (a) de dimérisation sélective du propylène dans laquelle le propylène est mis en contact avec une composition catalytique comprenant au moins un composé du nickel (Constituant A) mélangé ou complexé avec au moins une phosphine tertiaire (Constituant B), au moins un hydrocarbure aromatique consistant au moins en partie en du mésitylène (Constituant C), au moins un milieu non-aqueux à caractère ionique (Constituant D) résultant du mélange d'au moins un halogénure d'ammonium quaternaire ou d'au moins un halogénure de phosphonium quaternaire (Constituant Y) avec au moins un halogénure d'aluminium (Constituant Z) et éventuellement au moins un composé organométallique de l'aluminium (Constituant T) ;
- cette étape de dimérisation est suivie d'une étape (b) de distillation du mélange formé, pour obtenir le 2,3-DMB-1 à une haute pureté.

Dans le procédé de l'invention, il est avantageux que l'hydrocarbure aromatique consistant au moins en partie en du mésitylène (Constituant C) soit avec le Constituant A, compté en atomes de nickel, dans un rapport molaire d'au moins 1:1.

### Les composés du nickel (constituant A)

Les composés du nickel utilisés dans les compositions catalytiques de l'invention sont par exemple le chlorure, le bromure, le sulfate, les carboxylates, (par exemple l'éthyl-2 hexanoate), les phénates et l'acétylacétonate. On peut également utiliser les complexes organométalliques du nickel contenant ou non des phosphines. Ces complexes de nickel sont utilisés en mélange avec une phosphine tertiaire. On peut également utiliser des complexes du nickel déjà complexés avec une phosphine tertiaire.

### Les phosphines tertiaires (constituant B)

Les phosphines utilisées en mélange avec (ou pour complexer) les composés de nickel selon l'invention sont, à titre d'exemples, la triisopropylphosphine, la tricyclohexylphosphine et la tricyclopentylphosphine.

Le rapport molaire entre la phosphine (constituant B) et le composé du nickel (constituant A), compté en atomes de nickel, est en général compris entre 10:1 et 0,1:1 et de préférence entre 5:1 et 0,5:1.

Le composé du nickel mélangé ou complexé avec au moins une phosphine tertiaire est choisi plus particulièrement parmi les complexes NiCl₂,2 pyridine en mélange avec au moins un équivalent de phosphine tertiaire fonctionnalisée ou de phosphite fonctionnalisé, le chlorure de nickel en mélange avec au moins un équivalent de dicyclopentylphosphinoéthyl-2 pyridine-4, l'acétate de nickel en mélange avec au moins un équivalent de dicyclopentylphosphinoéthyl-2 pyridine-4, l'octoate (éthyl-2 hexanoate) de nickel en mélange avec au moins un équivalent de dicyclopentylphosphinoéthyl-2 pyridine-4 et le chlorure de π-allyl-nickel dicyclopentylphosphinoéthyl-2 pyridine-4.

### Les hydrocarbures aromatiques (constituant C)

Outre le mésitylène, d'autres hydrocarbures aromatiques peuvent être utilisés dans le procédé de l'invention. Ce sont des hydrocarbures aromatiques liquides à température ambiante, par exemple le benzène et ses substitués de formule générale C₆HₓR₆₋ₓ, R étant un radical alkyle, cycloalkyle, aryle, alkylaryle tel que C₆H₅CH₂-, et x prenant les valeurs de 1 à 5, autres que le mésitylène.

Ces hydrocarbures aromatiques pourront être utilisés comme solvants pour introduire la phosphine tertiaire.

### Le liquide ionique (constituant D)

Le milieu de type « liquide ionique » dans lequel est dissous le composé de nickel mélangé ou complexé avec au moins une phosphine tertiaire et au moins un hydrocarbure aromatique est constitué par mélange :
a) d'au moins un halogénure, plus particulièrement chlorure et/ou bromure, d'ammonium quaternaire et/ou phosphonium quaternaire (constituant Y) ;
b) d'au moins un halogénure d'aluminium (constituant Z) ; et
c) éventuellement d'au moins un composé organométallique d'aluminium (constituant T).

### Les halogénures d'ammonium quaternaires et les halogénures de phosphonium quaternaires (constituant Y)

Les halogénures d'ammonium quaternaires et les halogénures de phosphonium quaternaires utilisables dans le cadre de l'invention répondent de préférence aux formules générales :

NR¹R²R³R⁴X et PR¹R²R³R⁴X,

où X représente Cl ou Br, R¹, R², R³ et R⁴, identiques ou différents, représentant chacun l'hydrogène, un groupement hydrocarboné, aliphatique (saturé ou insaturé) ou aromatique, comprenant de 1 à 12 atomes de carbone. Les halogénures d'ammonium et/ou de phosphonium quartenaires peuvent également être dérivés d'hétérocycles comportant 1, 2 ou 3 atomes d'azote et/ou de phosphore. A titre d'exemples, on peut citer le chlorure de tétrabutylphosphonium, le chlorure de N-butylpyridinium, le bromure d'éthylpyridinium, le chlorure de butyl-3 méthyl-1 imidazolium, le chlorure de diéthylpyrazolium, le chlorhydrate de pyridinium et le chlorure de triméthylphénylammonium, le chlorure de butylméthylpyrrolidinium.

### Les halogénures d'aluminium (constituant Z)

Les halogénures d'aluminium utilisés selon l'invention sont essentiellement le chlorure et le bromure d'aluminium.

### Les composés organométalliques d'aluminium (constituant T)

Les composés organométalliques d'aluminium, facultatifs selon l'invention, ont pour formule générale AlRₓX₃₋ₓ dans laquelle R est un reste alkyle, linéaire ou ramifié, comportant de 2 a 8 atomes de carbone, X étant le chlore ou le brome et x ayant une valeur égale à 1, 2 ou 3.

Ces composés organométalliques d'aluminium sont introduits de façon facultative dans le liquide ionique. Ils peuvent aussi être ajoutés de façon indépendante au liquide ionique, de façon continue ou discontinue, par injection dans la section réactionnelle. Ils permettent d'activer le sel de nickel et de générer *in situ,* dans la phase réactionnelle, l'espèce active nickel-carbone ou nickel-hydrure pour dimériser le propylène.

A titre d'exemples, on peut utiliser le sesquichlorure d'isobutylaluminium, le sesquichlorure d'éthylaluminium, le dichloroisobutylaluminium, le dichloroéthylaluminium et le chlorodiéthylaluminium.

Les constituants du « liquide ionique » tels que définis ci-dessus sont en général mis en jeu dans les rapports molaires suivants :
- le Constituant Z est mis en jeu avec le Constituant Y dans un rapport molaire de 0,05:1 à 3:1, de préférence de 1:1 à 2:1 ;
- le Constituant T, facultatif, est mis en jeu dans un rapport molaire avec le Constituant Z au plus égal à 100:1, de préférence de 0.005:1 à 10:1.

Il est néanmoins nécessaire que les constituants et leurs proportions soient tels que le mélange soit liquide à la température à laquelle se fait l'introduction du composé du nickel et la phosphine tertiaire et de l'hydrocarbure aromatique, bien que la réaction catalytique de dimérisation puisse se faire à une température inférieure ou supérieure à la température de fusion de la composition catalytique.

De plus, quand on met en jeu un composé organométallique de l'aluminium (Constituant T), il est en général avec le Constituant A, compté en atomes de nickel, dans un rapport molaire compris entre 1:1 et 100:1, et de préférence compris entre 5:1 et 50:1.

### Introduction des constituants du système catalytique

Les composés entrant dans la composition catalytique selon l'invention peuvent être mélangés dans un ordre quelconque. Le mélange peut se faire par une simple mise en contact suivie d'une agitation jusqu'à formation d'un liquide homogène. Ce mélange peut être fait en dehors du réacteur de dimérisation ou, de préférence, dans ce réacteur.

Dans une mise en oeuvre en continu, le composé du nickel, la phosphine tertiaire et l'hydrocarbure aromatique peuvent être ajoutés en continu dans le réacteur afin de maintenir constantes les performances du système catalytiques (activité, sélectivité en dimères, sélectivité en 2,3-diméthylbut-1-ène).

### L'oléfine

Le propylène soumis à la dimérisation sélective de l'invention peut être utilisé pur ou dilué par un alcane tel qu'on le trouve dans les « coupes » C3 issues des procédés de raffinage du pétrole, comme le craquage catalytique ou le craquage a la vapeur.

### La réaction : conditions et mise en oeuvre

La réaction catalytique de dimérisation du propylène mise en oeuvre dans le procédé de l'invention peut être conduite en système fermé, en système semi-ouvert ou en continu, avec un ou plusieurs étages de réaction. Une vigoureuse agitation doit assurer un bon contact entre le (ou les) réactif(s) et le mélange catalytique. La température de réaction peut être de -40 à +70 °C, de préférence de -20 à +50 °C. On peut opérer au-dessus ou en dessous de la température de fusion du milieu, l'état solide dispersé n'étant pas une limitation au bon déroulement de la réaction. La chaleur engendrée par la réaction peut être éliminée par tous les moyens connus de l'homme du métier. La pression peut aller de la pression atmosphérique à 20 MPa, de préférence de la pression atmosphérique à 5 MPa.

Les produits de la réaction et le (ou les) réactif(s) qui n'ont pas réagi sont séparés du système catalytique par simple décantation, puis fractionnés.

Le mélange d'hydrocarbures obtenu contient une haute teneur en 2,3-diméthylbut-1-ène. Cependant, la séparation de cet isomère par distillation demeure délicate. Le 2,3-diméthylbut-1-ène a un point d'ébullition (55,2°C) très proche de ceux de deux autres isomères, le 4-méthylpent-1-ène (53,87°C) et le 4-méthylpent-2-ène *cis* (56,4°C).

Cette séparation par distillation peut néanmoins être envisagée si l'on opère avec une colonne à distiller présentant un nombre suffisant de plateaux de séparation. On a pu montrer au laboratoire que, en mode de fonctionnement batch et à partir du mélange issu directement du réacteur, une colonne possédant plus de 90 plateaux (nombre déterminé par l'efficacité de la séparation d'un mélange n-heptane/méthylcyclohexane) permet d'obtenir une fraction majoritaire contenant au moins 95 % de 2,3-diméthylbut-1-ène avec un rendement supérieur à 85 % de ce composé. On peut même atteindre 97 % de pureté dans l'isomère recherché si on limite cette récupération en dessous de 60 %. Ceci est évidemment obtenu en écartant les fractions les plus légères et les plus lourdes du mélange précédent.

Une stratégie industrielle pour permettre une séparation suffisante par la technique de distillation peut être développée. Dans cette perspective, une colonne unique en deux passes permet effectivement d'obtenir une pureté et un taux de récupération convenables.

On décrit ci-après un mode de réalisation de la distillation en continu, où les détails opératoires sont donnés à titre indicatif.

La colonne utilisée est alimentée en continu. Elle présente un nombre de plateaux qui atteint 100 (ce nombre est toujours déterminé expérimentalement par la séparation d'un mélange n-heptane/méthylcyclohexane.

Dans un premier temps on récupère en opérant avec un taux de reflux (reflux/distillat) égal à 60 une fraction de tête représentant environ 5 % de la charge et qui contient majoritairement déjà du 2,3-diméthylbut-1-ène avec la totalité du 4-méthylpent-1-ène.

Le fond de cette distillation est alors repris et traité dans la même colonne en maintenant un taux de reflux (reflux/distillat) de 20. La fraction de tête qui contient 75 % de 2,3-diméthylbut-1-ène présent dans la charge initiale présente une pureté de 95 % dans cet isomère. L'impureté principale est alors le 4-méthylpent-2-ène *cis,* mais des isomères plus lourds sont aussi déjà présents.

Si, dans la même stratégie, on a pris le soin de se débarrasser d'abord par distillation de la fraction constituée des fractions les plus lourdes (trimères du propylène) et supérieurs, on peut encore améliorer le taux de récupération qui alors peut atteindre jusqu'à 80 % du 2,3-diméthylbut-1-ène présent dans la charge avec une pureté qui atteint alors 96 % ; le 4-méthylpent-2-ène *cis* reste toutefois l'impureté majoritaire dans l'isomère recherché.

Bien entendu, l'homme du métier est en mesure d'effectuer toute l'opération en utilisant un train de distillations comprenant en premier lieu une colonne d'équeutage de la coupe d'hydrocarbures issue de l'opération de dimérisation/oligomérisation du propylène. Cette première colonne (I), dite de "re-run", qui n'a qu'une efficacité de séparation limitée (moins de 20 plateaux théoriques à partir des données de séparation du mélange n-heptane/méthylcyclohexane) permet d'éliminer en fond 90 % poids des composés C9+ avec un minimum de perte en hexènes (moins de 2 % poids), ces pertes ne contenant que des traces de l'isomère recherché, le 2,3-diméthylbut-1-ène, mais malheureusement une bonne partie de l'autre isomère di-ramifié, le 2,3-diméthylbut-2-ène.

La colonne (II) suivante permet l'étêtage déjà décrit ci-dessus et permet l'élimination totale du 4-méthylpent-1-ène avec on l'a vu une petite partie de 2,3-diméthylbut-1-ène. Le fond de la colonne (II) fournit la charge à une colonne (III) géométriquement identique à la colonne (II) et qui fonctionne aussi comme décrit ci-dessus. Cette colonne fournit alors en tête la fraction riche en 2,3-diméthylbut-1-ène avec une pureté au moins égale à 97 % avec un taux de récupération global qui peut atteindre 80 %.

Il faut noter que la fraction de tête de la colonne (II) et la fraction lourde de la colonne (III) peuvent être réunies. Cette nouvelle fraction est ensuite retraitée dans le même train des colonnes (II) et (III), ce qui permet une récupération supplémentaire comprise entre 4 et 6 % (calculée à partir du mélange initial) de l'isomère recherché avec une pureté à peine inférieure (95 %).

De la même façon, la réunion de ces deux fractions peut être valorisée par isomérisation (hydro-isomérisation) du 2,3-diméthylbut-1-ène en 2,3-diméthylbut-2-ène, suivant un processus connu de l'homme du métier, comme cela est indiqué dans le brevet US-A-4 835 328. Ce mélange est alors soumis à une opération de distillation qui sépare facilement l'isomère le plus lourd parmi les hexènes, à savoir le 2,3-diméthylbut-2-ène.

Les exemples suivants illustrent l'invention.

### Exemple 1 : Préparation du solvant ionique de composition (BMIC:AlCl₃:EtAlCl₂) (rapport molaire 1:1,22:0,22)

On a mélangé à température ambiante 10,06 g (57,3 mmoles) de chlorure de butyl-1 méthyl-3 imidazolium (BMIC), 9,35 g (70,04 mmoles) de chlorure d'aluminium et 1,58 g (12,44 mmoles) de dichloroéthylaluminium. On obtient ainsi un liquide.

### Exemple 2 : Préparation de la Solution N°1

Dans un tube de Schlenk, on introduit sous courant d'argon, 45,15 mg d'une solution d'octoate (éthyl-2 hexanoate) de nickel contenant 13 % en poids de nickel, soit 5,87 mg (0,099 mmole) de nickel ; 28 mg (0,1 mmole) de tricyclohexylphosphine ; 0,25 g (0,29 mL) de mésitylène, puis 20 mL d'heptane. On obtient une solution verte.

### Exemple 3 : Dimérisation du propylène

Un réacteur en verre à double étage, muni d'une sonde de mesure de température et d'un barreau aimanté dans l'étage du bas (de volume 20 mL), pour assurer une bonne agitation, et d'une double enveloppe permettant la circulation d'un liquide de réfrigération, a été purgé d'air et d'humidité et maintenu à la pression atmosphérique de propylène à 99 % de pureté, préalablement séché sur tamis moléculaire, par l'intermédiaire d'un détendeur à gaz liquéfié. On y a introduit l'ensemble de la Solution N°1 préparée dans l'Exemple 2 (contenant 0,1 mmole de Ni et 0,1 mmole de phosphine), puis on a abaissé la température à -15 °C et on a injecté à l'aide d'une seringue 5 mL (6,15 g) de la composition liquide préparée dans l'Exemple 1. On a mis l'agitation en route et on a observé immédiatement une absorption de propylène. Quand l'étage supérieur non agité a été plein de liquide, on a soutiré la plus grande partie de la phase hydrocarbonée. Au bout de 5 heures et 40 minutes (3 soutirages) on avait produit 22,6 kg de produits par gramme de Ni. Les phases organiques soutirées sont analysées par chromatographie en phase gazeuse sur colonne PONA (20°C, palier 20 min et montée de 10°C/min jusqu'à 270°C). Le Tableau 2 indique la composition des différentes fractions.

### Analyse des différentes fractions

**Tableau 2**

| N° de fraction | Oléfines C6/ produits totaux formés (% poids) | Distribution des isomères en C6 | | | | | | | | | |
|---|---|---|---|---|---|---|---|---|---|---|---|
| | | (% poids) | | | | | | | | | |
| | | M4P1 | 2,3-DMB-1 | M4P2c | M4P2t | M2P1 | H3t | H2t | M2P2 | H2c | 2,3-DMB-2 |
| 1 | 78,7 | 3,74 | 83,34 | 2,55 | 0,71 | 7,28 | 0 | 0,37 | 0,87 | 0,77 | 0,36 |
| 2 | 79,2 | 3,80 | 83,28 | 2,55 | 0,97 | 6,91 | 0,08 | 0,32 | 0,89 | 0,77 | 0,43 |
| 3 | 79,6 | 3,58 | 79,69 | 2,48 | 3,57 | 6,50 | 0,23 | 0,85 | 1,55 | 0,83 | 0,71 |

La composition des dimères comporte entre 80 et 83 % de 2,3-diméthylbut-1-ène.

### Exemple 4 : Préparation de la Solution N°2

De la même manière que dans l'Exemple 2, on réalise une solution de nickel contenant 1,49 g d'une solution d'octoate (éthyl-2 hexanoate) de nickel contenant 13 % en poids de nickel, soit 193,7 mg (3,3 mmoles) de nickel; 924 mg (3,3 mmoles) de tricyclohexylphosphine (PCy₃) ; 9,6 mL de mésitylène puis 208 mL d'heptane. On obtient une solution verte d'un volume total de 220 mL, dans laquelle les rapports molaires des constituants Ni/PCy₃/mésitylène sont : 1:1:20,9.

### Exemple 5 : Préparation d'une solution de dichloroéthylaluminium (Solution N°3)

Dans un tube de Schlenk sous argon, on introduit 18 mL de dichloroéthylaluminium (168,7 mmoles Al) et 18 mL d'heptane.

### Exemple 6 : Dimérisation du propylène : "test longue durée" - Stabilité des performances par rajouts discontinus des Solutions N°2 et N°3.

On opère comme dans l'Exemple 3, mais dans un réacteur en verre mono-étage d'une capacité unitaire d'environ 1 litre, muni d'une double enveloppe permettant la circulation d'un liquide de réfrigération, d'un barreau aimanté permettant l'agitation, et d'une sonde de température. Le réacteur est maintenu sous pression atmosphérique constante de propylène par l'intermédiaire d'un détendeur à gaz liquéfié (28 g). Il est refroidi à -15°C. On introduit alors successivement 20 mL de la Solution N°2 préparée dans l'Exemple 4 (contenant 0,3 mmole de nickel); et 15 mL (18,45 g) d'une solution de solvant ionique préparée selon l'Exemple 1. On démarre l'agitation. On observe immédiatement une absorption de propylène. Lorsque le réacteur est plein de liquide, on soutire à l'aide d'une canule en verre, plongeant dans une zone calme du réacteur, la phase organique surnageante que l'on analyse par chromatographie en phase gazeuse comme dans l'Exemple 3.

Afin de maintenir constantes les performances de la réaction (vitesse de réaction, sélectivité en 2,3-DMB-1 et sélectivité en hexènes), on ajoute à l'aide d'une seringue, après chaque soutirage, une fraction de la solution décrite dans l'Exemple 4 (Solution N°2) et une fraction de la solution décrite dans l'Exemple 5 (Solution N°3) (Tableau 3, ci-après).

**Tableau 3**

| N° de fraction | Temps | Poids de produit formé | 2,3-DMB-1/ C6 | C6/ produits | Solution N°2 ajoutée | Solution N°2 ajoutée | Solution N°3 ajoutée |
|---|---|---|---|---|---|---|---|
| | (h:min) | (g) | (%pds) | (%pds) | (mL) | (mmol Ni) | (mmol Al) |
| Démarrage | | | | | | 0,3 | 11,1 |
| 1 | 04:15 | 422,1 | 83 | 78 | 20 | 0,3 | 11,1 |
| 2 | 06:35 | 773,22 | 82,6 | 75,5 | 0 | 0 | 0 |
| 3 | 08:19 | 1018,08 | 77,5 | 77 | 10 | 0,15 | 5,55 |
| 4 | 11:45 | 1335,6 | 76 | 80 | 20 | 0,3 | 11,1 |
| 5 | 13:40 | 1656,48 | 78,5 | 79 | 10 | 0,15 | 5,55 |
| 6 | 15:26 | 1974 | 77,4 | 77 | 5 | 0,07 | 2,77 |
| 7 | 17:23 | 2282,28 | 75 | 79 | 5 | 0,07 | 2,77 |
| 8 | 19:55 | 2623,32 | 71,3 | 79 | 10 | 0,15 | 5,55 |
| 9 | 22:20 | 2965,2 | 70,2 | 79,3 | 5 | 0,07 | 2,77 |

On a donc produit avec même liquide ionique (15 mL) après 22 heures et 20 minutes de réaction 3 kg de produit, composé de 77-80 % d'hexènes contenant une moyenne de 77 % de 2,3-DMB-1.

### Exemple 7 : Préparation d'une solution de tricyclohexylphosphine dans le mésitylène (Solution N°4)

Dans un tube de Schlenk, on introduit sous courant d'argon 0,7 g (2,5 mmoles) de tricyclohexylphosphine, 7,22 mL (51,9 mmoles) de mésitylène et 20 mL d'heptane. Le volume total de la solution est de 28 mL.

### Exemple 8 : Préparation d'une solution de nickel dans le mésitylène (Solution N°5)

Dans un tube de Schlenk, on introduit sous courant d'argon 2,03 g d'une solution d'octoate (éthyl-2 hexanoate) de nickel (soit 4,4 mmoles de nickel), 13 mL (93,4 mmoles) de mésitylène et 60 mL d'heptane. Le volume total de la solution est 75 mL.

### Exemple 9 : Dimérisation du propylène : "test longue durée", avec rajout de nickel et de PCy₃ directement dans le mésitylène.

On poursuit l'Exemple 6, mais, après avoir soutiré la fraction 9, on rajoute une fraction de la Solution N°5 de nickel et de la Solution N°4 de PCy₃.

La sélectivité en 2,3-DMB-1 remonte ainsi doucement (72 % par rapport aux C6 totaux), ainsi que la sélectivité en dimères (81,3 %/produits).

**Tableau 4**

| N° de fraction | Temps (h:min) | Poids de produit formé | 2,3-DMB-1/ C6 | C6/ produits | Solution N°5 ajoutée | Solution N°4 ajoutée | Solution N°3 ajoutée |
|---|---|---|---|---|---|---|---|
| | | (g) | (% pds) | (% pds) | (mmol Ni) | (mmole de PCy₃) | (mmol Al) |
| 11 | 28:04 | 3714,5 | 72 | 81,3 | 0,15 | 0,75 | 5,55 |

Le test selon l'Exemple 6 a été poursuivi pendant plus de 40 heures (Tableau 5). On a recueilli plus de 6 kg de produits avec 15 mL de liquide ionique.

**Tableau 5**

| N° de fraction | Temps | Poids de produit | 2,3-DMB-1/ C6 | C6/ produits | Solution N°2 ajoutée | Solution ajoutée N°3 |
|---|---|---|---|---|---|---|
| | (h:min) | (g) | (% poids) | (% poids) | (mmol Ni) | (mmol Al) |
| 12 | 29:55 | 4031,16 | 75,7 | 78,9 | 0,15 | 5,55 |
| 13 | 31:50 | 4483,92 | 76,4 | 76,4 | 0,15 | 5,55 |
| 14 | 33:50 | 4798,92 | 75,6 | 79,7 | 0,15 | 5,55 |
| 15 | 35:45 | 5118,12 | 75,8 | 79,7 | 0,15 | 5,55 |
| 16 | 37:51 | 5429,76 | 75,7 | 78 | 0,15 | 5,55 |
| 17 | 39:54 | 5724,6 | 73,9 | 80,7 | 0,15 | 5,55 |
| 18 | 41:41 | 6003,48 | 72,8 | 74,3 | 0,15 | 5,55 |
| 19 | 43:41 | 6317,64 | 75,6 | 79,5 | 0,15 | 5,55 |

### Exemple 10

On donne ici les résultats de la microdistillation en mode "batch" d'un mélange d'hexènes obtenus par dimérisation de propylène suivant la procédure de la présente invention.

Cet exemple montre que l'on peut obtenir directement par distillation une quantité notable de 2,3-diméthylbut-1-ène à partir d'un mélange réactif brut préalablement équeuté.

La colonne utilisée est une colonne de microdistillation qui permet l'opération en batch d'environ 50 mL du mélange réel à séparer.

Cette colonne se caractérise par le fait qu'on y a déterminé 90 plateaux théoriques lors de la séparation d'un mélange n-heptane/méthylcyclohexane.

La colonne est tout d'abord mise à reflux total doux puis on commence le prélèvement en tête avec un taux de reflux d'environ 50, des portions de distillat de 1 mL.

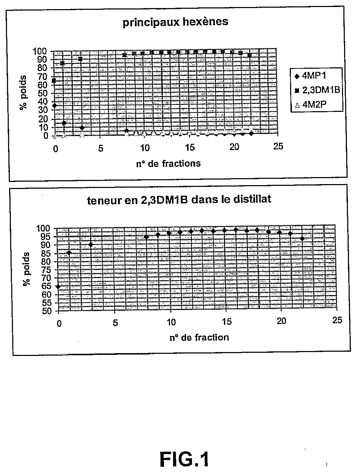

Les diagrammes donnés à la Figure 1 montrent que la concentration en 2,3-diméthylbut-1-ène est supérieure à 95 % poids dans les fractions 10 à 21. Les fractions 22 à 43 contiennent encore plus de 80 % poids de l'isomère intéressant.

On s'aperçoit ainsi qu'il est relativement facile de se débarrasser du 4-méthylpent-1-ène, mais que l'impureté gênante est le 4-méthylpent-2-ène *cis.* Cela conduit à recycler dans une autre opération les fractions 1 à 9 de cette distillation primaire qui sont riches en 2,3-diméthylbut-1-ène et singulièrement pauvres en 4-méthylpent-2-ène *cis* en mélange avec la charge brute pour améliorer encore la récupération en isomère intéressant et porter la récupération finale en 2,3-diméthylbut-1-ène à plus 60 % avec une pureté nettement supérieure à 95 % poids mais qui reste cependant inférieure à 97 % poids, l'impureté majoritaire restant le 4-méthyl pent-2-ène *cis.*

### Exemple 10 bis

Dans cet exemple, on recycle avec la charge les fractions 22 à 40 de la distillation primaire et on obtient dans les conditions expérimentales mises en jeu dans le distillat une pureté supérieur à 97,5 % poids mais alors, la récupération en isomère intéressant, le 2,3-diméthylbut-1-ène, n'atteint qu'au maximum 55 % poids.

### Exemple 11

On utilise cette fois une colonne au pouvoir séparateur supérieur à celle décrite dans l'Exemple 9, puisque, avec un mélange n-heptane/méthylcyclohexane, on a pu y mesurer au minimum 100 plateaux théoriques.

Cette colonne est alimentée en continu par le mélange d'hexènes déjà utilisé précédemment. On s'organise pour instaurer un taux de reflux (reflux/distillat) compris entre 40 et 60 avec un ratio distillat sur charge de 0,04.

Dans ces conditions, on détermine par chromatographie en phase gazeuse que la teneur en 4-méthylpent-1-ène dans le distillat atteint au moins 70 % poids, le reste étant constitué majoritairement de 2,3-diméthylbut-1-ène, avec moins de 0,5 % poids de 4-méthylpent-2-ène *cis.*

Dans une deuxième passe sur la même colonne mais avec un taux de reflux inférieur à 20 et en s'arrangeant pour se placer dans des conditions où le ratio distillat sur charge fraîche est d'environ 0,65, on récupère un distillat en tête de colonne une fraction qui contient majoritairement et à plus de 95 % poids l'isomère recherché, le 2,3-diméthylbut-1-ène, avec une pureté de 97 % poids.

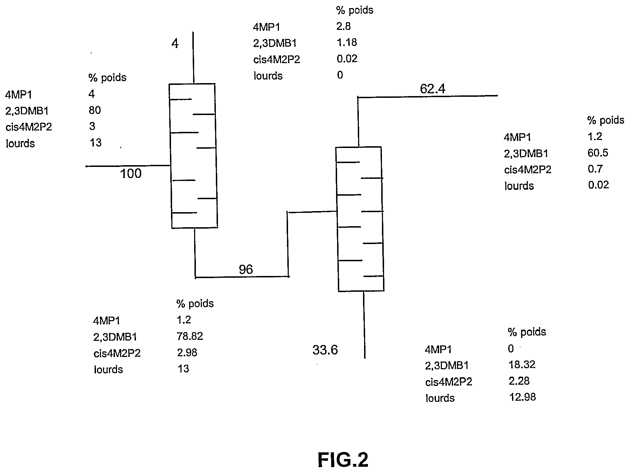

Le schéma de la Figure 2 illustre cet exemple. Sur ce schéma, les deux passes ont été représentées sur deux colonnes différentes et on a indiqué les compositions :
- du mélange d'hexènes à purifier ;
- des distillats et résidu de première passe ; et
- des distillats et résidu de seconde passe.

## Revendications

1. Procédé de fabrication de 2,3-diméthylbut-1-ène, noté 2,3-DMB-1, **caractérisé en ce qu'**il comprend une étape de dimérisation sélective du propylène dans laquelle le propylène est mis en contact avec une composition catalytique comprenant au moins un composé du nickel, noté Constituant A, mélangé ou complexé avec au moins une phosphine tertiaire, noté Constituant B, au moins un hydrocarbure aromatique consistant au moins en partie en du mésitylène, noté Constituant C, au moins un milieu non-aqueux à caractère ionique, noté Constituant D, résultant du mélange d'au moins un halogénure d'ammonium quaternaire ou d'au moins un halogénure de phosphonium quaternaire, noté Constituant Y, avec au moins un halogénure d'aluminium, noté Constituant Z, et éventuellement au moins un composé organométallique de l'aluminium, noté Constituant T et une étape de distillation du mélange formé, pour obtenir le 2,3-DMB-1 à haute pureté.

2. Procédé selon la revendication 1 **caractérisé en ce que** le Constituant C comprend, outre le mésitylène, au moins un autre hydrocarbure aromatique liquide à température ambiante choisi parmi le benzène et ses substitués de formule générale C₆HₓR₆₋ₓ, R étant un radical alkyle, cycloalkyle, aryle, alkylaryle et x prenant les valeurs de 1 à 5.

3. Procédé selon l'une des revendications 1 ou 2 **caractérisé en ce que** le Constituant C est avec le Constituant A, compté en atomes de nickel, dans un rapport molaire d'au moins 1:1.

4. Procédé selon l'une des revendications 1 à 3 **caractérisé en ce que** le Constituant A est choisi parmi le chlorure, le bromure, le sulfate, les carboxylates, les phénates et l'acétylacétonate.

5. Procédé selon l'une des revendications 1 à 4 **caractérisé en ce que** le composé du nickel mélangé ou complexé avec au moins une phosphine tertiaire est choisi parmi les complexes NiCl₂,2 pyridine en mélange avec au moins un équivalent de phosphine tertiaire fonctionnalisée ou de phosphite fonctionnalisée le chlorure de nickel en mélange avec au moins un équivalent de dicyclopentylphosphinoéthyl-2 pyridine-4, l'acétate de nickel en mélange avec au moins un équivalent de dicyclopentylphosphinoéthyl-2 pyridine-4, l'octoate (éthyl-2 hexanoate) de nickel en mélange avec au moins un équivalent de dicyclopentylphosphinoéthyl-2 pyridine-4 et le chlorure de π-allyl-nickel dicyclopentylphosphinoéthyl-2 pyridine-4.

6. Procédé selon l'une des revendications 1 à 5 **caractérisé en ce que** le rapport molaire entre la phosphine (constituant B) et le composé du nickel (constituant A), compté en atomes de nickel, est compris entre 10:1 et 0,1:1.

7. Procédé selon l'une des revendications 1 à 6 **caractérisé en ce que** l'halogénure d'ammonium quaternaire ou l'halogénure de phosphonium quaternaire utilisable comme Constituant Y dans ledit milieu non-aqueux à caractère ionique répond à la formule générale :
NR¹R²R³R⁴X ou PR¹R²R³R⁴X,
où X représente Cl ou Br, R¹, R², R³ et R⁴, identiques ou différents, représentent chacun l'hydrogène, un groupement hydrocarboné, aliphatique, saturé ou insaturé, ou aromatique, comprenant de 1 à 12 atomes de carbone, ou dérivent d'hétérocycles comportant 1, 2 ou 3 atomes d'azote et/ou phosphore.

8. Procédé selon l'une des revendications 1 à 7 **caractérisé en ce que** l'halogénure d'aluminium utilisable comme Constituant Z dans ledit milieu non-aqueux à caractère ionique est le chlorure ou le bromure d'aluminium.

9. Procédé selon l'une des revendications 1 à 8 **caractérisé en ce que**, dans ledit milieu non-aqueux à caractère ionique, le Constituant Z est mis en jeu avec le Constituant Y dans un rapport molaire de 0,05:1 à 3:1.

10. Procédé selon l'une des revendications 1 à 9 **caractérisé en ce que** le milieu non aqueux à caractère ionique comprend en outre un Constituant T comprenant au moins un composé organométallique de l'aluminium répondant à la formule générale AlRₓX₃₋ₓ, dans laquelle R est un reste alkyle, linéaire ou ramifié, comportant de 2 à 8 atomes de carbone, X étant le chlore ou le brome et x a une valeur égale à 1, 2 ou 3.

11. Procédé selon l'une des revendications 10 **caractérisé en ce que** le Constituant T est mis en jeu dans un rapport molaire avec le Constituant Z au plus égal à 100:1.

12. Procédé selon l'une des revendications 10 ou 11 **caractérisé en ce que** le Constituant T est mis en jeu dans un rapport molaire avec le Constituant A, compté en atomes de nickel, au plus égal à 100:1.

13. Procédé selon l'une des revendications 1 à 12 **caractérisé en ce que** le propylène est contenu dans une « coupe » C3 issue d'un procédé de raffinage du pétrole.

14. Procédé selon l'une des revendications 1 à 13 **caractérisé en ce que** ladite distillation est réalisée en batch.

15. Procédé selon l'une des revendications 1 à 13 **caractérisé en ce que** ladite distillation est réalisée en continu en deux passes sur une même colonne.

## Patentansprüche

1. Verfahren zur Herstellung von 2,3-Dimethylbut-1-en, bezeichnet als 2,3-DMB-1, **dadurch gekennzeichnet, dass** dieses umfasst: einen Schritt der selektiven Dimerisation von Propylen, wobei das Propylen mit einer katalytischen Zusammensetzung in Berührung gebracht wird, umfassend mindestens eine Nickel-Verbindung, bezeichnet als Bestandteil A, gemischt oder komplexiert mit mindestens einem tertiären Phosphin, bezeichnet als Bestandteil B, mindestens einem aromatischen Kohlenwasserstoff, der mindestens teilweise aus Mesitylen besteht, bezeichnet als Bestandteil C, mindestens einem nicht-wässrigen Medium mit ionischem Charakter, bezeichnet als Bestandteil D, der resultiert aus der Mischung mindestens eines quaternären Ammoniumhalogenids oder mindestens eines quaternären Phosphoniumhalogenids, bezeichnet als Bestandteil Y, mit mindestens einem Aluminiumhalogenid, bezeichnet als Bestandteil Z, und gegebenenfalls mindestens einer Aluminiumorganometall-Verbindung, bezeichnet als Bestandteil T, und einen Schritt der Destillation der gebildeten Mischung, um 2,3-DMB-1 mit hoher Reinheit zu erhalten.

2. Verfahren nach Anspruch 1, **dadurch gekennzeichnet, dass** der Bestandteil C, abgesehen von Mesitylen, mindestens einen anderen aromatischen Kohlenwasserstoff umfasst, der bei Umgebungstemperatur flüssig ist, ausgewählt aus Benzol und seinen Substituenten der allgemeinen Formel C₆HₓR₆₋ₓ, wobei R ein Alkyl-, Cycloalkyl-, Aryl-, Alkylarylrest ist, und x Werte von 1 bis 5 annimmt.

3. Verfahren nach einem der Ansprüche 1 oder 2, **dadurch gekennzeichnet, dass** der Bestandteil C mit dem Bestandteil A, gezählt in Nickelatomen, in einem Molverhältnis von mindestens 1:1 steht.

4. Verfahren nach einem der Ansprüche 1 bis 3, **dadurch gekennzeichnet, dass** der Bestandteil A aus Chlorid, Bromid, Sulfat, Carboxylaten, Phenaten und Acetylacetonat ausgewählt ist.

5. Verfahren nach einem der Ansprüche 1 bis 4, **dadurch gekennzeichnet, dass** die Nickel-Verbindung, gemischt oder komplexiert mit mindestens einem tertiären Phosphin, ausgewählt ist aus NiCl₂,2-Pyridin-Komplexen, in Mischung mit mindestens einem funktionalisierten tertiären Phosphin- oder funktionalisierten Phosphit-Äquivalent, Nickelchlorid in Mischung mit mindestens einem 2-Dicyclopentylphosphinoethyl-4-pyridin-Äquivalent, Nickelacetat in Mischung mit mindestens einem 2-Dicyclopentylphosphinoethyl-4-pyridin-Äquivalent, Nickeloctoat (2-Ethylhexanoat) in Mischung mit mindestens einem 2-Dicyclopentylphosphinoethyl-4-pyridin-Äquivalent, und π-Allylnickel-2-dicyclopentylphosphinoethyl-4-pyridinchlorid-Äquivalent.

6. Verfahren nach einem der Ansprüche 1 bis 5, **dadurch gekennzeichnet, dass** das Molverhältnis zwischen Phosphin (Bestandteil B) und der Nickel-Verbindung (Bestandteil A), gezählt in Nickelatomen, zwischen 10:1 und 0,1:1 liegt.

7. Verfahren nach einem der Ansprüche 1 bis 6, **dadurch gekennzeichnet, dass** das quaternäre Ammoniumhalogenid oder das quaternäre Phosphoniumhalogenid, das als Bestandteil Y in dem nicht-wässrigen Medium mit ionischem Charakter verwendbar ist, die allgemeine Formel aufweist:
NR¹R²R³R⁴X oder PR¹R²R³R⁴X,
wobei X Cl oder Br bedeutet, R¹, R², R³ und R⁴, die identisch oder verschieden sind, jeweils Wasserstoff, eine aliphatische, gesättigte oder ungesättigte, oder aromatische Kohlenwasserstoffgruppe mit 1 bis 12 Kohlenstoffatomen bedeuten oder von Heterozyklen mit 1, 2 oder 3 Stickstoff- und/oder Phosphoratomen stammen.

8. Verfahren nach einem der Ansprüche 1 bis 7, **dadurch gekennzeichnet, dass** das Aluminiumhalogenid, das als Bestandteil Z in dem nicht-wässrigen Medium mit ionischem Charakter verwendbar ist, Aluminiumchlorid oder -bromid ist.

9. Verfahren nach einem der Ansprüche 1 bis 8, **dadurch gekennzeichnet, dass** in dem nicht-wässrigen Medium mit ionischem Charakter der Bestandteil Z mit dem Bestandteil Y in einem Molverhältnis von 0,05:1 bis 3:1 verwendet wird.

10. Verfahren nach einem der Ansprüche 1 bis 9, **dadurch gekennzeichnet, dass** das nicht-wässrige Medium mit ionischem Charakter ferner einen Bestandteil T umfasst, umfassend mindestens eine Aluminiumorganometall-Verbindung der allgemeinen Formel AlRₓX₃₋ₓ, wobei R ein linearer oder verzweigter Alkylrest mit 2 bis 8 Kohlenstoffatomen ist, wobei X Chlor oder Brom ist, und x einen Wert gleich 1, 2 oder 3 hat.

11. Verfahren nach Anspruch 10, **dadurch gekennzeichnet, dass** der Bestandteil T in einem Molverhältnis mit dem Bestandteil Z von höchstens gleich 100:1 verwendet wird.

12. Verfahren nach einem der Ansprüche 10 oder 11, **dadurch gekennzeichnet, dass** der Bestandteil T in einem Molverhältnis mit dem Bestandteil A, gezählt in Nickelatomen, von höchstens gleich 100:1 verwendet wird.

13. Verfahren nach einem der Ansprüche 1 bis 12, **dadurch gekennzeichnet, dass** das Propylen in einem "Schnitt" C3 enthalten ist, der aus einem Erdölraffinationsverfahren stammt.

14. Verfahren nach einem der Ansprüche 1 bis 13, **dadurch gekennzeichnet, dass** die Destillation als Batchdestillation durchgeführt wird.

15. Verfahren nach einem der Ansprüche 1 bis 13, **dadurch gekennzeichnet, dass** die Destillation kontinuierlich in zwei Passagen auf derselben Säule durchgeführt wird.

## Claims

1. A process for the preparation of 2,3-dimethylbut-1-ene, referred to as 2,3-DMB-1, **characterised in that** it comprises a step for selective dimerisation of propylene, in which the propylene is brought into contact with a catalytic composition comprising at least one compound of nickel, referred to as constituent A, which is mixed or complexed with at least one tertiary phosphine, referred to as constituent B, at least one aromatic hydrocarbon consisting at least in part of mesitylene, referred to as constituent C, at least one non-aqueous medium of ionic nature, referred to as constituent D, resulting from mixing at least one quaternary ammonium halide or at least one quaternary phosphonium halide, referred to as constituent Y, with at least one aluminium halide, referred to as constituent Z, and optionally at least one organometallic compound of aluminium referred to as constituent T, and a step for distillation of the mixture formed to give 2,3-DMB-1 in a state of high purity.

2. A process according to claim 1 **characterised in that** besides the mesitylene the constituent C comprises at least one other aromatic hydrocarbon which is liquid at ambient temperature selected from benzene and its substitutes of the general formula C₆HₓR₆₋ₓ, R being an alkyl, cycloalkyl, aryl or alkylaryl radical and x assuming the values of 1 to 5.

3. A process according to one of claims 1 and 2 **characterised in that** the constituent C is in a molar ratio of at least 1:1 with the constituent A considered as nickel atoms.

4. A process according to one of claims 1 to 3 **characterised in that** the constituent A is selected from chloride, bromide, sulphate, carboxylates, phenates and acetylacetonate.

5. A process according to one of claims 1 to 4 **characterised in that** the compound of nickel which is mixed or complexed with at least one tertiary phosphine is selected from the complexes NiCl₂,2 pyridine mixed with at least one equivalent of functionalised tertiary phosphine or functionalised phosphite, nickel chloride mixed with at least one equivalent of dichloropentyl phosphino 2-ethyl 4-pyridine, nickel acetate mixed with at least one equivalent of dichloropentyl phosphino 2-ethyl 4-pyridine, nickel octoate (2-ethyl hexanoate) mixed with at least one equivalent of dichloropentyl phosphino 2-ethyl 4-pyridine and the chloride of π-allyl-nickel dichloropentyl phosphino 2-ethyl 4-pyridine.

6. A process according to one of claims 1 to 5 **characterised in that** the molar ratio between the phosphine (constituent B) and the nickel compound (constituent A), reckoned as nickel atoms, is between 10:1 and 0.1:1.

7. A process according to one of claims 1 to 6 **characterised in that** the quaternary ammonium halide or the quaternary phosphonium halide which can be used as constituent Y in said non-aqueous medium of ionic nature corresponds to the general formula:
NR¹R²R³R⁴X or PR¹R²R³R⁴X,
wherein X represents Cl or Br, R¹, R², R³ and R⁴ which are identical or different each represent hydrogen, an aliphatic, saturated or unsaturated, or aromatic hydrocarbon group comprising from 1 to 12 of carbon atoms or derived from heterocycles comprising 1, 2 or 3 nitrogen and/or phosphorus atoms.

8. A process according to one of claims 1 to 7 **characterised in that** the aluminium halide which can be used as constituent Z in said non-aqueous medium of ionic nature is aluminium chloride or bromide.

9. A process according to one of claims 1 to 8 **characterised in that** in said non-aqueous medium of ionic nature the constituent Z is used with the constituent Y in a molar ratio of from 0.05:1 to 3:1.

10. A process according to one of claims 1 to 9 **characterised in that** the non-aqueous medium of ionic nature further comprises a constituent T comprising at least one organometallic compound of aluminium corresponding to the general formula AlRₓX₃₋ₓ, in which R is a straight-chain or branched alkyl radical comprising from 2 to 8 carbon atoms, X being chlorine or bromine and x being of a value equal to 1, 2 or 3.

11. A process according to claim 10 **characterised in that** the constituent T is used in a molar ratio with the constituent Z that is at most equal to 100:1.

12. A process according to one of claims 10 and 11 **characterised in that** the constituent T is used in a molar ratio with the constituent A reckoned as nickel atoms that is at most equal to 100:1.

13. A process according to one of claims 1 to 12 **characterised in that** the propylene is contained in a C3 "cut" resulting from a petroleum refining process.

14. A process according to one of claims 1 to 13 **characterised in that** said distillation operation is implemented batchwise.

15. A process according to one of claims 1 to 13 **characterised in that** said distillation operation is implemented continuously in two passes on the same column.
